(19) Europäisches Patentamt
European Patent Office
Office européen des brevets

(11) Veröffentlichungsnummer : **0 128 437**
**B1**

(12) # EUROPÄISCHE PATENTSCHRIFT

(45) Veröffentlichungstag der Patentschrift : **02.03.88**

(51) Int. Cl.⁴ : **C 07 D451/06**, C 07 D451/10

(21) Anmeldenummer : **84106030.4**

(22) Anmeldetag : **26.05.84**

(54) **Neues Nortropinderivat, Verfahren zu seiner Herstellung und seine Verwendung.**

(30) Priorität : **03.06.83 DE 3320138**

(43) Veröffentlichungstag der Anmeldung : **19.12.84 Patentblatt 84/51**

(45) Bekanntmachung des Hinweises auf die Patenterteilung : **02.03.88 Patentblatt 88/09**

(84) Benannte Vertragsstaaten : **AT BE CH DE FR GB IT LI NL SE**

(56) Entgegenhaltungen :
**DE-A- 2 026 661**
**DE-A- 2 540 633**
**Arzneimittelforschung 12, 305 (1962)**
**Die Akte enthält technische Angaben, die nach dem Eingang der Anmeldung eingereicht wurden und die nicht in dieser Patentschrift enthalten sind.**

(73) Patentinhaber : **BOEHRINGER INGELHEIM KG**
**D-6507 Ingelheim am Rhein (DE)**
**AT BE CH DE FR GB IT LI NL SE**
**Boehringer Ingelheim International G.m.b.H**

**D-6507 Ingelheim am Rhein (DE)**
**GB**

(72) Erfinder : **Banholzer, Rolf, Dr.**
**Johann-Calvin-Strasse 11**
**D-6507 Ingelheim am Rhein (DE)**

EP 0 128 437 B1

Anmerkung : Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

Jouve, 18, rue St-Denis, 75001 Paris, France

**Beschreibung**

Die Erfindung betrifft die neue Verbindung N-β-Fluorethylnortropin der Formel I

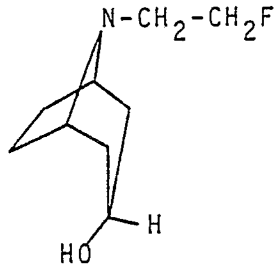

(I)

ihre Säureadditionssalze, Verfahren zu ihrer Herstellung sowie ihre Weiterverarbeitung zu Pharmazeutika.

Das N-β-Fluorethylnortropin ist ein wertvolles Zwischenprodukt, beispielsweise zur Herstellung des pharmazeutisch wertvollen Benzilsäure-N-β-fluorethylnortropinesters und dessen Quartärsalzen der allgemeinen Formel II,

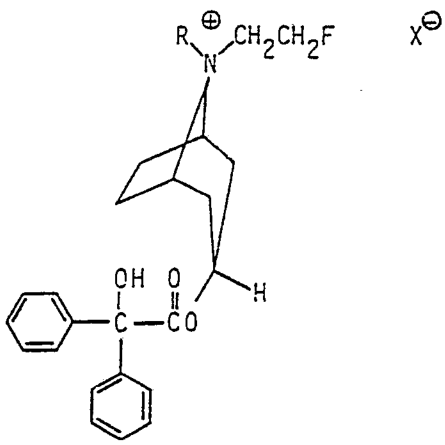

(II)

worin R = H und einen geradkettigen oder verzweigten Alkylrest, vorzugsweise Methylrest und $X^{\ominus}$ ein pharmakologisch unbedenkliches Anion, wie ein Halogenatom oder den Rest einer organischen Sulfonsäure darstellt.

Die Verbindungen der Formel II sind ihrerseits wertvolle Pharmazeutika mit anticholinerger Wirkung. Sie eignen sich hervorragend für die Behandlung von Spasmen und Bronchospasmen. Diese Substanzen sind u. a. in der DE-OS 25 40 633 beschrieben.

Aus der DE-OS 20 26 661 ist bekannt, daß N-Alkylnortropine durch Veresterung mit substituierten Essigsäuren und Quaternierung in Arzneistoffen übergeführt werden können. Es gibt jedoch keinen Hinweis in der Anmeldung, daß auch N-Halogenalkyl- bzw. N-Fluoralkylnortropine eingesetzt werden können, besonders wertvolle Ausgangsstoffe darstellen und wie sie hergestellt werden können.

In Arzneimittelforschung 12, S.305-309 (1962) werden N-Alkylnortropine und ihre Herstellung beschrieben und auf die Schwierigkeit hingewiesen, sterisch einheitliche Verbindungen zu erhalten. Zur Herstellung der N-Alkylnortropan-3α-ole wird Hydrierung bei ca. 50 bar in Ethanol vorgeschlagen. N-Halogenalkylderivate sind nicht erwähnt. Es muß allerdings angenommen werden, daß N-Halogenalkylnortropine unter den Reaktionsbedingungen nicht ausreichend stabil sind. Auch bei der Einführung der β-Fluorethylgruppe waren beträchtliche Nebenreaktionen zu erwarten, da die Reaktivität z. B. des Broms im 1-Brom-2-fluorethan herabgesetzt ist und nach der Verknüpfung mit dem Stickstoff in Gegenwart der stark basischen Substanzen mit der Abspaltung von Fluorwasserstoff und der Bildung von Bis-Verbindungen gerechnet werden mußte.

Die Herstellung des N-β-Fluorethylnortropins erfolgt dabei so, daß man

a) Nortropin bzw. dessen Salze, hergestellt nach literaturbekannten Verfahren, z. B. T. D. Perrine J. Org. Chem. 16, 1303 (1951) S. P. Findlay J. Amer. chem. Soc. 75, 3204 (1953) und G. Kraiss und K. Nádor Tetrahedron Letters (London) 1971, (1) 57 mit Verbindungen der allgemeinen Formel IIa

$$Y{-}CH_2{-}CH_2{-}F \qquad \text{(IIa)}$$

wobei Y eine sog. « leaving group », vorzugsweise ein Halogenatom bedeutet, alkyliert.

Insbesondere verwendet man 2-Bromfluorethan, welches nach F. L. M. Pattison und W. C. Howell J.

Org. Chem. 21, 748 (1956) herstellbar ist.

Hierbei werden die üblichen Bedingungen der N-Alkylierung eines sekundären Amins eingehalten.

b) Ein weiteres Verfahren zur Herstellung der Verbindung der allgemeinen Formel I besteht in der selektiven Hydrierung von N-β-Fluorethylnortropinon zum 3-α-Ol mit geeigneten Katalysatoren wie Raney-Nickel. Das hierzu erforderliche N-β-Fluorethylnortropinon kann entweder durch Alkylierung von Nortropinon bzw. dessen Salzen mit 2-Halogenfluorethanen wie 2-Bromfluorethan oder durch Robinson-Schöpf-Kondensation mit 2-Aminofluorethan gewonnen werden.

Die Überführung in Säureadditionssalze kann in üblicher Weise durch Umsetzung der Base der Formel I mit Säuren (z. B. Salzsäure, Bromwasserstoffsäure, Schwefelsäure, Oxalsäure etc.) erfolgen.

Die Weiterverarbeitung der Verbindung der Formel I zu den bereits literaturbekannten (Benzilsäure-N-β-fluorethylnortropinester (s. DE-OS 25 40 633) führt über die Acylierung des N-β-Fluorethylnortropins. Dies kann beispielsweise mittels Benzilsäure-Imidazolid in üblicher Weise erfolgen (s. Beispiel 2).

Mit üblichen Quaternierungsmitteln der allgemeinen Formel III

$$RX \qquad (III)$$

wobei R und X die oben genannte Bedeutung haben, erhält man daraus die Quartärverbindungen des Benzilsäure-N-β-fluorethylnortropinesters der allgemeinen Formel II, wie in der DE-OS 25 40 633 beschrieben.

Soll die Verbindung der Formel I oder ihre physiologisch verträglichen Säureadditionssalze allein oder in Kombination mit anderen Substanzen als pharmazeutischer Wirkstoff verwendet werden, so erfolgt ihre Verarbeitung zu Pharmazeutika wie in der DE-OS 25 40 633 (S. 7/8 bzw. 20-24) beschrieben.

Die folgenden Beispiele erläutern die Erfindung.

Beispiel 1 (Herstellung)

N-β-Fluorethylnortropin, isoliert als N-β-Fluorethylnortropinhydrochlorid

33,8 g (0,265 7 Mol) Nortropin, 37,1 g (0,292 2 Mol) 2-Bromfluorethan und 31,0 g (0,292 2 Mol) Natriumcarbonat werden in 300 ml absolutem Acetonitril 7,5 Stunden unter Rühren und unter Rückfluß erhitzt. Danach werden die anorganischen Salze abgesaugt, mit Acetonitril gewaschen und die gesammelten Mutterlaugen eingeengt. Den öligen Rückstand löst man in Methylenchlorid und stellt auf übliche Art und Weise mit Chlorwasserstoffgas das N-β-Fluorethylnortropin-hydrochlorid her. Weiße Kristalle (Isopropanol), Schmp. : 184-186 °C (Zers.)

Ausbeute : 48,6 g (87,2 % d. Th.).

Beispiel 2 (Weiterverarbeitung)

Benzilsäure-N-β-fluorethylnortropinester, isoliert als Benzilsäure-N-β-fluorethylnortropinester-hydrochlorid

Zu einer siedenden Lösung von 5,2 g (0,03 Mol) wasserfreiem N-β-Fluorethylnortropin in 50 ml absolutem Aceton werden 8,4 g (0,03 Mol) Benzilsäureimidazolid in vier Anteilen und in Zeitabständen von etwa 15 Min. gegeben. Man läßt die entstandene Suspension unter Rückflußbedingungen weiterreagieren. Falls die Umsetzung noch nicht ausreichend ist, wird ein weiterer Anteil von Benzilsäureimidazolid zugegeben, wobei wie beschrieben verfahren wird.

Nach einer Gesamtreaktionszeit von 51 Stunden wird das Aceton abdestilliert, der ölige Rückstand in Methylenchlorid aufgenommen und ausgiebig erst mit destilliertem Wasser, dann mit verdünnter Salzsäure extrahiert. Die vereinigten sauren Wasserphasen werden alkalisch gestellt und das Umsetzungsprodukt durch Extraktion mit Methylenchlorid gewonnen. Die vereinigten Methylenchloridextrakte werden über Natriumsulfat getrocknet, nach dem Abdestillieren des Methylenchlorids wird der zurückbleibende Rückstand auf übliche Art und Weise mit Chlorwasserstoffgas zum Benzilsäure-N-β-fluorethylnortropinester-hydrochlorid umgesetzt.

Weiße Kristalle (Methanol-Ether, Acetonitril)

Schmp. : 205-206 °C (Zers.)

Ausbeute 5,1 g (40,5 % d. Th.)

**Patentansprüche**

1. N-β-Fluorethylnortropin der Formel I

(Siehe Formel I Seite 4 f.)

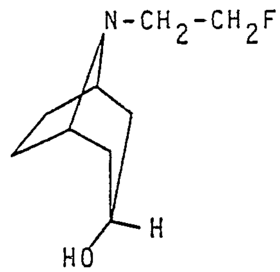

(I)

und dessen Säureadditionssalze.

2. Verfahren zur Herstellung von N-β-Fluorethylnortropin und dessen Salze, dadurch gekennzeichnet, daß man Nortropin bzw. seine Salze mit einer Verbindung der allgemeinen Formel IIa

$$Y{-}CH_2{-}CH_2{-}F \quad \text{(IIa)}$$

wobei Y eine sog. « leaving group » bedeutet, alkyliert.

3. Verfahren nach Anspruch 2, dadurch gekennzeichnet, daß man als Verbindung der Formel IIa das 2-Bromfluorethan einsetzt.

4. Verfahren zur Herstellung von N-β-Fluorethylnortropin und dessen Salzen, dadurch gekennzeichnet, daß man N-β-Fluorethylnortropinon bzw. seine Salze selektiv mit $H_2$/Raney-Nickel zum N-β-Ethylnortropan-3α-ol reduziert.

5. Verwendung von N-β-Fluorethylnortropin oder dessen Salze zur Herstellung von Pharmazeutika.

6. Verwendung von N-β-Fluorethylnortropin zur Herstellung von pharmazeutischen Wirkstoffen der allgemeinen Formel II

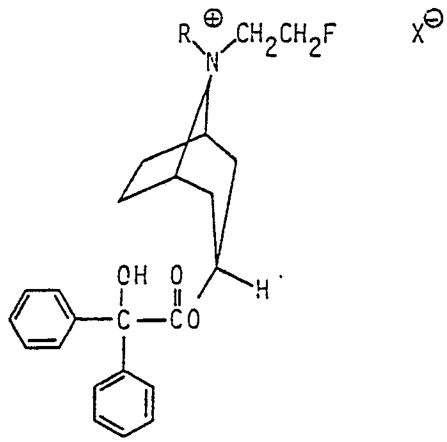

(II)

worin R einen geradkettigen oder verzweigten Alkylrest, vorzugsweise Methylrest und $X^{\ominus}$ ein pharmakologisch unbedenkliches Anion, ein Halogenatom oder den Rest einer organischen Sulfonsäure darstellt.

**Claims**

1. N-β-Fluorethylnortropine of formula I

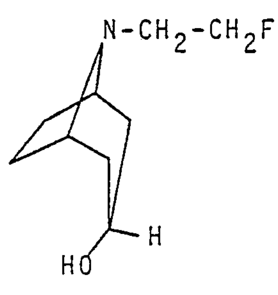

(I)

and the acid addition salts thereof.

2. Process for preparing N-β-fluorethylnortropine and the salts thereof, characterised in that nortropine or a salt thereof is alkylated with a compound of general formula IIa

$$Y{-}CH_2{-}CH_2{-}F \quad \text{(IIa)}$$

wherein Y represents a so-called leaving group.

3. Process as claimed in claim 2, characterised in that 2-bromofluoroethane is used as the compound of formula IIa.

4. Process for preparing N-β-fluorethylnortropine and the salts thereof, characterised in that N-β-fluorethylnortropinone or a salt thereof is selectively reduced with $H_2$/Raney nickel to obtain the N-β-ethylnortropan-3α-ol.

5. Use of N-β-fluorethylnortropine or a salt thereof for the preparation of pharmaceutical compositions.

6. Use of N-β-fluorethylnortropine for the preparation of pharmaceutically active substances of general formula II

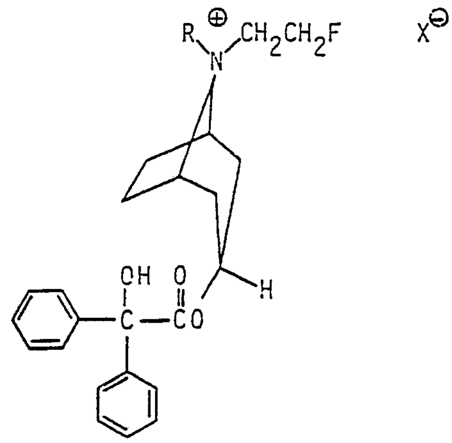

(II)

wherein R represents a straight-chained or branched alkyl group, preferably a methyl group and $X^-$ represents a pharmacologically harmless anion, a halogen atom or the residue of an organic sulphonic acid.

**Revendications**

1. N-β-fluoréthylnortropine répondant à la formule I

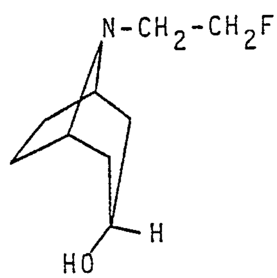

(I)

et ses sels d'addition aux acides.

2. Procédé pour la préparation de la N-β-fluoréthylnortropine et de ses sels, caractérisé en ce qu'on alcoyle la nortropine ou ses sels avec un composé répondant à la formule générale IIa

$$Y{-}CH_2{-}CH_2{-}F \quad \text{(IIa)}$$

dans laquelle Y désigne un « groupe partant ».

3. Procédé suivant la revendication 2, caractérisé en ce qu'on utilise comme composé répondant à la formule IIa le 2-bromofluoréthane.

4. Procédé pour la préparation de la N-β-fluoréthylnortropine et de ses sels, caractérisé en ce qu'on réduit sélectivement la N-β-fluoréthylnortropinone ou ses sels avec du $H_2$/nickel de Raney pour donner le N-β-éthyl-nortropane-3α-ol.

5. Utilisation de la N-β-fluoréthylnortropine ou de ses sels pour la préparation de produits pharmaceutiques.

6. Utilisation de la N-β-fluoréthylnortropine pour la préparation de substances pharmaceutiques actives répondant à la formule générale II

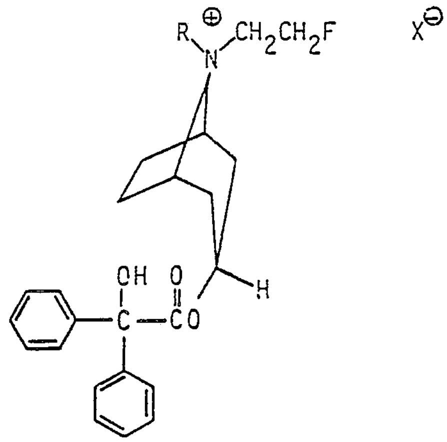

(II)

dans laquelle R représente un radical alcoyle linéaire ou ramifié, de préférence le radical méthyle et $X^-$ un anion pharmacologiquement sans danger, un atome d'halogène ou le radical d'un acide sulfonique organique.